(19) Europäisches Patentamt
European Patent Office
Office européen des brevets

(11) Numéro de publication: **0 214 881 B1**

# (12) FASCICULE DE BREVET EUROPEEN

(45) Date de publication du fascicule du brevet: **27.09.89**

(51) Int. Cl.⁴: **A61K 31/62**

(21) Numéro de dépôt: **86401592.0**

(22) Date de dépôt: **17.07.86**

(54) **Compositions pharmaceutiques à base de diltiazem et d'aspirine.**

(30) Priorité: **30.07.85 FR 8511603**

(43) Date de publication de la demande:
**18.03.87 Bulletin 87/12**

(45) Mention de la délivrance du brevet:
**27.09.89 Bulletin 89/39**

(84) Etats contractants désignés:
**BE CH DE FR GB IT LI LU NL SE**

(56) Documents cités:
**CHEMICAL ABSTRACTS, vol. 99, no. 7, 15 août 1983, résumé no. 47687p, Columbus, Ohio, US; A. KIYOMOTO et al.: "Inhibition of platelet aggregation by diltiazem. Comparison with verapamil and nifedipine and inhibitory potencies of diltiazem metabolites", & CIRC. RES., SUPPL. 1983, 52(1), 115-19 000**
**CHEMICAL ABSTRACTS, vol. 102, no. 5, 4 février 1985, résumé no. 39699j, Columbus, Ohio, US; H.L. LIPPTON et al.: "Influence of verapamil and diltiazme on aggregatory responses in cat and rabbit platelet rich plasma", & PROSTAGLANDINS, LEUKOTRIENES MED. 1984, 16(1), 121-30 1983, 52(1), 115-19 000**

(73) Titulaire: **SYNTHELABO, 58, rue de la Glacière, F-75621 Paris Cedex 13(FR)**

(72) Inventeur: **Langer, Salomon Z., 92, rue Jouffroy, F-75017 Paris(FR)**

(74) Mandataire: **Thouret-Lemaitre, Elisabeth et al, Service Brevets - SYNTHELABO 58, rue de la Glacière, F-75621 Paris Cédex 13(FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 214 881 B1

ACTORUM AG

## Description

La présente invention a pour objet des compositions pharmaceutiques à base de diltiazem et d'aspirine, plus particulièrement d'acétylsalicylate de lysine, destinées au traitement de l'agrégation plaquettaire.

Le diltiazem de formule

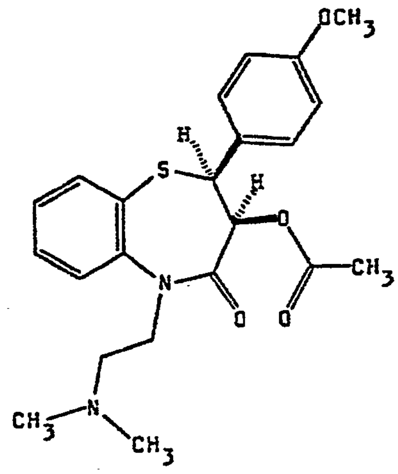

(1)

est connu pour ses propriétés antiangineuses, antihypertensives, antagonistes du calcium et antiagrégantes plaquettaires.

L'acétylsalicylate de lysine (ASL) de formule

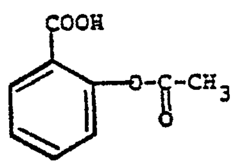 , $NH_2-(CH_2)_4CH(NH_2)COOH$

est connu pour ses propriétés analgésiques, antiinflammatoires et antiagrégantes plaquettaires.

La Demanderesse a constaté que, de manière surprenante, il existe une potentialisation des propriétés antiagrégantes plaquettaires de chacun des composés lorsqu'on associe les deux composés.

Les compositions pharmaceutiques de l'invention ont été soumises à une série d'essais pharmacologiques.

Les compositions ont été testées in vitro de la manière suivante:
les composés sont administrés soit seul, soit en association, à une suspension de plaquettes et de plasma contenue dans les bacs de 250 ul d'un agrégomètre, deux minutes avant l'addition de 10 ug d'acide arachidonique, agent inducteur de l'agrégation plaquettaire.

On suit l'agrégation dans un agrégomètre chronologue à 2 canaux selon la méthode de Born, Journal of Physiology, 168, 178-195 (1963).

Les résultats obtenus sont donnés dans les tableaux 1 et 2.

Tableau 1

| Concentration de ASL µM | Concentration de diltiazem µM | % inhibition de l'agrégation plaquettaire | | |
|---|---|---|---|---|
| | | ASL seul | diltiazem seul | ASL + diltiazem |
| 10 | 200 | 6 | 3 | 88 |
| 20 | 200 | 13 | 12 | 66 |
| 50 | 200 | 25 | 9 | 72 |
| 50 | 200 | 9 | 6 | 79 |
| 20 | 400 | 7 | 27 | 96 |
| 50 | 400 | 9 | 27 | 100 |

ASL = acétylsalicylate de lysine.

Tableau 2

| Concentration de diltiazem µM | Concentration de ASL µM | % inhibition de l'agrégation plaquettaire | | |
|---|---|---|---|---|
| | | diltiazem seul | ASL seul | diltiazem + ASL |
| 200 | 10 | 3 | 6 | 88 |
| 200 | 20 | 12 | 13 | 66 |
| 400 | 20 | 27 | 7 | 96 |
| 200 | 50 | 9 | 25 | 72 |
| 400 | 50 | 27 | 9 | 100 |

Les compositions de l'invention ont été testées in vivo de la manière suivante:
le diltiazem à une dose de 10 mg/kg et l'ASL à une dose de 1 mg/kg sont administrés par voie orale, soit seul, soit en association, à cinq groupes de deux rats. Au bout d'une heure, des échantillons de sang sont prélevés. On prépare alors des suspensions de plasma et de plaquettes que l'on place dans les bacs de 250 µl de l'agrégomètre deux minutes avant l'addition d'acide arachidonique (200-250 µg/ml). On suit l'agrégation dans un agrégomètre chronologue à 2 canaux selon la méthode de Born.

Les résultats sont donnés dans le tableau 3.

Tableau 3

| Dose d'ASL mg/kg p. o. | Dose de diltiazem mg/kg p. o. | % inhibition de l'agrégation plaquettaire (moyenne de 5 expériences) | | |
|---|---|---|---|---|
| | | ASL seul | Diltiazem seul | ASL + Diltiazem |
| 1 | 10 | 8 | 9 | 81 |

Les résultats montrent que l'association d'acétylsalicylate de lysine et de diltiazem produit un effet antiagrégant plaquettaire significativement plus important que la somme des effets engendrés par chacun des composés administrés isolément.

Il y a potentialisation de l'activité antiagrégante plaquettaire de chacun des composés, ASL et diltiazem, par l'autre.

Les compositions pharmaceutiques de l'invention peuvent contenir de 3 à 60 mg de diltiazem et de 10 à 300 mg d'ASL par unité de prise.

Les compositions pharmaceutiques de l'invention peuvent être présentées sous toute forme appropriée pour l'administration par voie orale ou parentérale, en association avec tout excipient approprié.

Les compositions pharmaceutiques de l'invention peuvent être utilisées pour le traitement de la thrombose coronaire et cérébrovasculaire, des atteintes vasculaires périphériques des troubles de l'agrégation plaquettaire et des migraines.

La posologie quotidienne peut être de 2 à 3 unités de prise.

**Revendications**

1. Composition pharmaceutique contenant une association de diltiazem et d'aspirine, plus particulièrement d'acétylsalicylate de lysine.

2. Composition pharmaceutique selon la revendication 1, caractérisée en ce qu'elle contient de 3 à 60 mg de diltiazem et de 10 à 300 mg d'acétylsalicylate de lysine, par unité de prise.

**Patentansprüche**

1. Pharmazeutische Zubereitung enthaltend eine Kombination aus Diltiazem und Aspirin, insbesondere Lysinacetylsalicylat.

2. Pharmazeutische Zubereitung nach Anspruch 1, dadurch gekennzeichnet, daß sie 3 bis 60 mg Diltiazem und 10 bis 300 mg Lysinacetylsalicylat pro Einnahmeeinheit enthält.

## Claims

1. Pharmaceutical composition containing a combination of diltiazem and aspirin, more especially lysine acetylsalicylate.

2. Pharmaceutical composition according to claim 1, characterized in that it contains from 3 to 60 mg of diltiazem and from 10 to 300 mg of lysine acetylsalicylate per unit dose.